# EUROPEAN PATENT SPECIFICATION

(11) **EP 1 365 765 B1**
(45) Date of publication and mention of the grant of the patent: **25.07.2007**
(21) Application number: 02722637.2
(22) Date of filing: 09.01.2002
(51) Int. Cl.: A61K 31/567, A61P 15/00

(54) **THE USE OF ANTIGESTAGENS FOR INHIBITING ACCELERATED ENDOMETRIAL MATURATION DURING INFERTILITY TREATMENT**
VERWENDUNG VON ANTIGESTAGENE ZUR VERSCHIEBUNG DER ENDOMETRIALEN REIFUNG WAHREND INFERTILITÄTBEHANDLUNG
UTILISATION D'ANTI-GESTAGENES POUR INHIBER UNE MATURATION ACCELEREE DE L'ENDOMETRE AU COURS D'UN TRAITEMENT CONTRE L'INFERTILITE

(30) Priority: 09.01.2001 US 756286; 09.03.2001 US 801925
(43) Date of publication of application: 03.12.2003
(73) Proprietor: Bayer Schering Pharma Aktiengesellschaft, 13353 Berlin (DE)
(72) Inventor: HEGELE-HARTUNG, Christa, 45470 Muehlheim a. d. Ruhr (DE); HESS-STUMPP, Holger, 13505 Berlin (DE); BEIER, Henning, M., 52076 Aachen (DE); KRUSCHE, Claudia, 52074 Aachen (DE)
(86) International application number: PCT/IB2002/001764
(87) International publication number: WO 2002/067910

(56) References cited:
- WO-A-00/39148
- WO-A-98/34947
- FUHRMANN U ET AL: "SYNTHESIS AND BIOLOGICAL ACTIVITY OF A NOVEL, HIGHLY POTENT PROGESTERONE RECEPTOR ANTAGONIST" JOURNAL OF MEDICINAL CHEMISTRY, AMERICAN CHEMICAL SOCIETY. WASHINGTON, US, vol. 43, no. 26, 2000, pages 5010-5016, XP001064233 ISSN: 0022-2623
- CHWALISZ K ET AL: "Antiproliferative effects of progesterone antagonists and progesterone receptor modulators on the endometrium" STEROIDS, BUTTERWORTH-HEINEMANN, STONEHAM, MA, US, vol. 65, no. 10-11, October 2000 (2000-10), pages 741-751, XP004224002 ISSN: 0039-128X
- SLAYDEN O D ET AL: "Progesterone antagonists increase androgen receptor expression in the rhesus macaque and human endometrium." THE JOURNAL OF CLINICAL ENDOCRINOLOGY AND METABOLISM. UNITED STATES JUN 2001, vol. 86, no. 6, June 2001 (2001-06), pages 2668-2679, XP001107002 ISSN: 0021-972X
- BEN-NUN I ET AL: "EFFECT OF PREOVULATORY PROGESTERONE ADMINISTRATION ON THE ENDOMETRIAL MATURATION AND IMPLANTATION RATE AFTER IN VITRO FERTILIZATION AND EMBRYO TRANSFER" FERTILITY AND STERILITY, ELSEVIER SCIENCE INC, NEW YORK, NY, US, vol. 53, no. 2, 1 February 1990 (1990-02-01), pages 276-281, XP002040088 ISSN: 0015-0282

## Description

### BACKGROUND OF THE INVENTION

This invention relates to the use of antigestagens for shifting post-ovulatory endometrial maturation during infertility treatment.

Ovarian stimulation with gonadotropins is commonly used in humans in assisted reproductive technologies, including in vitro fertilization and embryo transfer therapy (IVF/ET). However, the post-ovulatory endometrial transformation is advanced after controlled ovarian hyperstimulation (Garcia et al., 1984, Fertil. Steril. 41:31-37; Paulson et al., 1997, Fertil. Steril. 76:321-325; Kolb, 1997, Fertil. Steril. 67:625-630; Franchin et al., 1999, Fertil. Steril. 71:174-181.) As a consequence, the usual precisely synchronized temporal development of the endometrium and the embryo is disrupted, resulting in low implantation rates for healthy blastocysts.

Fuhrmann et al., 2000, J. Med. Chem., 43 (26):5010-5016; Chwalisz et al., 2000, Steroids, 65 (10-11):741-751 and WO98/34947 disclose the preparation of compounds of formula IA, IB and IC (vide infra), and suggest their use in medicine for the treatment of breast cancer, leyomiomas, dysmenorrhoea, endometriosis and for hormone replacement therapy. None of these documents discloses the use of these compounds to inhibit the occurrence of endometrium maturation in subjects undergoing infertility treatment and for achieving pregnancy.

Ben-Nun et al., 1990, Fertil. Steril.,53 (2):276-281 discloses the use of progesterone to promote the implantation rate after in-vitro fertilization.

### SUMMARY OF THE INVENTION

This invention provides the use of at least one compound of formula I 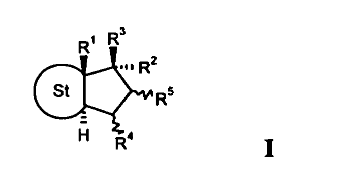 wherein
- R¹: is methyl or ethyl,
- R²: is CₙFₘHₒ, wherein n is 1-6, preferably 2, 3, 4, 5 or 6, m > 1 and m+o=2n+ 1,
- R³: is a free, etherified or esterified hydroxyl group,
- R⁴ and R⁵: each is hydrogen, or together form and additional bond or a methylene group,
- St: is a steroidal ABC-ring system of partial formula A, B or C 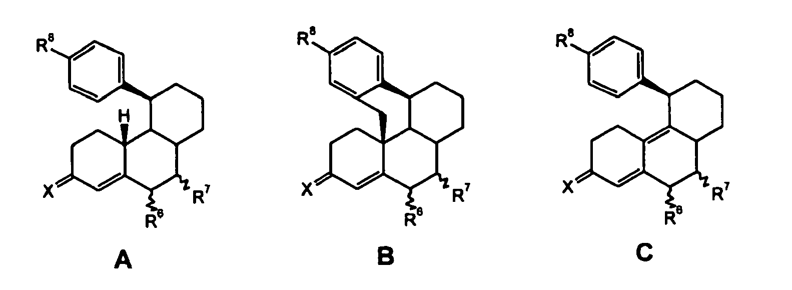
wherein
- R⁶: is hydrogen, a straight-chain C₁-C₄ alkyl group or branched C₃-C₄ alkyl group or halogen,
- R⁷: is hydrogen, a straight-chain C₁-C₄ alkyl group or a branched C₃-C₄ alkyl group, or if St is a steroidal ABC-ring system A or B, in addition
- R⁶ and R⁷: together can form an additional bond,
- X: is oxygen, hydroxyimino (=N-OH) or two hydrogen atoms,
- R⁸: is Y or aryl that is optionally substituted in several places with a group Y, other than H,
- Y: is hydrogen, halogen, -OH, -NO₂, -N₃, -CN, -NR^{9a}R⁹b, -NHSO₂R⁹, -CO₂R⁹, C₁-C₁₀alkyl, C₁-C₁₀alkoxy, C₁C₁₀alkanoyloxy, benzoyloxy, C₁-C₁₀alkanoyl, C₁-C₁₀hydroxyalkyl or benzoyl,
- R^{9a} and R^{9b}: are the same or different and each is hydrogen or C₁-C₁₀ alkyl,
- R⁹: is hydrogen or C₁-C₁₀alkyl,
and for -NR^{9a}R^{9b} radicals,as well as their physiologically compatible salts with acids and for -CO₂R⁹ radicals with R⁹ being hydrogen, as well as their physiologically compatible salts with bases, for production of a medicament for inhibiting the occurrence of advanced endometrium maturation in a human female subject undergoing fertility enhancing treatment, including all assisted reproduction therapies.

It is also contemplated as part of this invention that such a use will be particularly useful in a female mammal undergoing fertility treatment. Fertility treatment, as used herein, refers to any treatment which is rendered to a female mammal for the purposes of achieving a pregnancy, whether or not the mammal has been determined to be fertile, infertile, or to have impaired fertility. Fertility treatments contemplated as part of this invention include, but are not limited to ovarian hyperstimulation (OH), in vitro fertilization and embryo transfer (IVF/ET), and OH in combination with IVF/ET. Particularly, the use in inhibiting advanced endometrial maturation, which typically occurs in conjunction with ovarian hyperstimulation, is contemplated. For example, ovarian hyperstimulation may be used as a tool in fertility treatment in conjunction with, for example, timed intercourse, artificial insemination, intrauterine insemination, IVF/ET, and gamete intra fallopian transfer (GIFT).

IVF/ET, as used herein, refers to any techniques wherein oocytes are fertilized in vitro and then transferred into the female reproductive tract. For example, transfer of the embryo may occur via transfer of the embryo into the uterus through the cervix or via transfer of the embryo into the fallopian tubes (zygote intra fallopian transfer (ZIFT)). Fertilization of oocytes in vitro may be accomplished, for example, by incubating oocytes in the presence of sperm or by intra cytoplasmic sperm injection (ICSI). IVF/ET may also be accomplished using donor eggs. Infertility treatments also include treatments such as induction of ovarian stimulation followed by fertilization by normal coitus.

Ovarian hyperstimulation, as used herein, refers to the use of a follicle stimulating agent to stimulate follicle development. Ovarian hyperstimulation may be used in female subjects having a variety of ovulatory conditions, including subjects who are anovulatory, subjects who have impaired, reduced, or irregular ovulation, and subjects with normal ovulatory patterns. Preferred follicle stimulating agents, include gonadotropins, such as for example, follicle stimulating hormone (FSH), luteinizing hormone (LH), human menopausal gonadotropins (hMG), and human chorionic gonadotropin (hCG). Brand names of gonadotropins that are available for use as follicle stimulating agents, include for example, Perganol, Metrodin, Humegon, Fertinorm, Gonal F, and Primogonyl-1000 etc. Follicle stimulating agents, as used herein, also include estrogen blocking agents such as, for example, clomiphene citrate (commercially available as Clomid and Serophene). Gonadotropins that may be used in accordance with this invention include hormones that are isolated from naturally occurring source materials and hormones that are produced synthetically, including hormones produced through recombinant DNA techniques. Mutant gonadotropins which retain activity as follicle stimulating agents are also contemplated as part of this invention.

Ovarian hyperstimulation may be accomplished using more than type of follicular stimulating agent. For example, an estrogen blocking agent such as clomiphene may be used in combination with a gonadotropin. Ovarian hyperstimulation may be used to treat a variety of types of infertility, including but not limited to, idiopathic infertility, anovulatory infertility, endometriosis associated infertility, tubal factor infertility and male factor infertility.

Ovarian hyperstimulation may also be accomplished in conjunction with a gonadotropin releasing hormone antagonist (GnRH) to turn off the subjects endogenous hormone production. GnRH's that may be used in conjunction with the invention include, for example, Synarel or Lupron.

In one aspect, the invention relates to the use of at least one compound of formula I in inhibiting the occurrence of advanced endometrium maturation in a human female subject undergoing fertility treatment comprising administering at least one 17α-fluoralkylated progesterone receptor antagonist of formula I to the female subject during the post-ovulatory phase of the endometrial cycle.

Preferably, the gonadotropin administered to the mammal to achieve ovarian hyperstimulation comprises follicle stimulating hormone (FSH). Even more preferably ovarian stimulation is achieved by the use of a first gonadotropin comprising FSH and the subsequent use of second gonadotropin comprising chorionic gonadotropin, particularly human chorionic gonadotropin (hCG). Preferably the embryo is introduced into the reproductive tract of the mammal by introduction into the uterus via the cervix. Also preferably, a gonadotropin releasing hormone (GnRH) agonist or antagonist is administered to the female mammal prior and during the administration of the gonadotropins.

The compounds of formula I useful as antigestagens according to the present invention represent 17α-fluoralkylated progesterone receptor antagonists which possess a strong affinity for the gestagen receptor (progesterone receptor) and show minimal gestagen activity of their own. The 17α-fluoralkylated steroidal compounds which may be employed in conjunction with the invention are described U.S. Patent Application Serial No. 09/020,947, WO/98/34947.

The wavy lines in formula I and in partial formulas A, B and C mean that the substituent in question can be in α- or β-position.

Suitable alkyl groups within the scope of this formula include methyl, ethyl, or n- or iso-propyl, and n-, iso- or tert-butyl groups.

The other C₁-C₁₀ alkyl groups, Y, R⁹, R^{9a}, R^{9b}, include the higher homologs in addition, such as, for example, the pentyl, neo-pentyl, and hexyl to decyl groups.

C₁-C₁₀ alkyl groups are to be understood to encompass, however, carbocyclic or alkylcycloalkyl groups as well with up to 10 carbon atoms, for example cyclopropyl, cyclopentyl, cycloheptyl, methylcyclopropyl, methylcyclopentyl or methylcyclohexyl. A methyl or ethyl group is preferred for all cases above.

C₁-C₁₀ alkoxy groups are the radicals that are lengthened by one oxygen atom and derived from the alkyl groups that are mentioned above, thus, e.g., the methoxy, ethoxy, n- or iso-propoxy, n-, iso- or tert-butoxy radical, etc.

C₁-C₁₀ alkanoyl is defined as the acyl radicals of straight-chain and branched C₁-C₁₀ alkanecarboxylic acids, thus, for example, the formyl, acetyl, propionyl, butyryl or iso-butyryl radical, etc.

C₁-C₁₀ alkanoyloxy radicals are the radicals of the above alkanoyl radicals that are lengthened by one oxygen atom, thus, e.g., the acetyloxy, propionyloxy, and butyryloxy, etc.

If a halogen atom is mentioned as a substituent, this can be a fluorine, chlorine or bromine atom. Fluorine is preferred.

For radicals R², perfluorinated side chains of length n = 2-4 are preferred and among the latter, in turn the pentafluoroethyl unit is especially preferred.

R³ stands primarily for a free hydroxy group.

In the case of an etherified or esterified hydroxy group as a 17β-substituent, the latter is preferably etherified with a C₁-C₁₀ alkyl group or esterified with a C₁-C₁₀ alkanoyl group. For this alkyl or alkanoyl group, the same meanings as above apply. The etherification or esterification of the hydroxy group is carried out according to the methods that are familiar to one skilled in the art.

R⁴ and R⁵ preferably each are a hydrogen or together form an additional bond.

If R⁸ is a group Y, this is preferably a C₁-C₁₀ alkanoyl or (1-hydroxy)-C₁-C₁₀ alkyl group; among these, acetyl and propionyl are especially preferred.

Preferred carbocyclic or heterocyclic aryl radicals are phenyl, 1- or 2-naphthalinyl, 2- or 3-furanyl, 2- or 3-benzofuranyl, 2- or 3-thienyl, 2-, 3- or 4-pyridinyl. Substituted aryl radicals R⁸, are primarily 4-cyanophenyl and 4-halophenyl, especially 4-fluorophenyl, can be cited.

Among all the radicals that are mentioned as preferred for R⁸, R⁸ in the meaning of Y and Y, in turn, equal to acetyl is especially to be preferred.

The compounds that are mentioned below are especially preferred according to the invention:
11β-(4-Acetylphenyl)-17β-hydroxy-17α-(1,1,2,2,2-pentafluoroethyl)estr-4-en-3-one;
4'-[17β-hydroxy-3-oxo-17α-(1,1,2,2,2-pentafluoroethyl)estr-4-en-11β-yl][1,1'-biphenyl]-4-carbonitrile;
11β-(4'-fluoro[1,1'-biphenyl]-4-yl)-17β-hydroxy-17α-(1,1,2,2,2-pentafluoroethyl)estr-4-en-3-one;
17β-hydroxy-17α-(1,1,2,2,2-pentafluoroethyl)-11β-[4-(3-pyridinyl)phenyl]estr-4-en-3-one;
11β-(4-acetylphenyl)-17β-hydroxy-17α-(1,1,2,2,2-pentafluoroethyl)estra-4,15-dien-3-one;
4'-[17β-hydroxy-3-oxo-17α-(1,1,2,2,2-pentafluoroethyl)estra-4,15-dien-11β-yl][1,1'-biphenyl]-4-carbonitrile;
11β-(4'-fluoro[1,1-biphenyl]-4-yl)-17β-hydroxy-17α-(1,1,2,2,2-pentafluoroethyl)estra-4,15-dien-3-one;
17β-hydroxy-17α-(1,1,2,2,2-pentafluoroethyl)-11β-[4-(3-pyridinyl)phenyl]estr-4,15-dien-3-one;
11β-(4-acetytphenyl)-17β-hydroxy-17α-(1,1,2,2,2-pentaftuoroethyl)estra-4,9-dien-3-one;
4'-[17β-hydroxy-3-oxo-17α-(1,1,2,2,2-pentafluoroethyl)estra-4,9-dien-11β-yl] [1,1'-biphenyl]-4-carbonitrile;
11β-(4'-fluoro[1,1'-biphenyl]-4-yl)-17β-hydroxy-17α-(1,1,2,2,2-pentafluoroethyl)estra-4, 9-dien-3-one;
17β-hydroxy-17α-(1,1,2,2,2-pentafluoroethyl)-11β-[4-(3-pyridinyl)phenyl]estra-4,9-dien-3-one;
11β-(4-acetylphenyl)-17β-hydroxy-17α-(1,1,2,2,2-pentafluoroethyl)estra-4,9,15-trien-3-one;
4'-[17β-hydroxy-3-oxo-17α-(1,1,2,2,2-pentafluoroethyl)estra-4,9,15-trien-11β-yl][1,1'-biphenyl]-4-carbonitrile;
11β-(4'-fluoro[1,1'-biphenyl]-4-yl)-17β-hydroxy-17α-(1,1,2,2,2-pentafluoroethyl))estra-4,9,15-trien-3-one;
17β-hydroxy-17α-(1,1,2,2,2-pentafluoroethyl)-11β-[4-(3-pyridinyl)phenyl]estra-4,9,15-trien-3-one;
6'-acetyl-9,11α-dihydro-17β-hydroxy-17α-(1,1,2,2,2-pentafluoroethyl)-4'H-naphth[3',2',1':10,9,11]estr-4-en-3-one;
4-[9,11α-dihydro-17β-hydroxy-3-oxo-17α-(1,1,2,2,2-pentafluoroethyl)-4'H-naphth[3',2',1':10,9,11]estr-4-en-6'-yl]benzonitrile;
9,11α-dihydro-6'-(4-fluorophenyl)-17β-hydroxy-17α-(1,1,2,2,2-pentafluoroethyl)-4'H-naphth[3',2' ,1': :10,9,11]estr-4-en-3-one ;
9,11α-dihydro-17β-hydroxy-17α-(1,1,2,2,2-pentafluoroethyl)-6'-(3-pyridinyl)-4' H-naphth[3',2',1': 10,9,11]estr-4-en-3-one ;
6'-acetyl-9,11α-dihydro-17β-hydroxy-17α-(1,1,2,2,2-pentafluoroethyl)-4' H-naphth[3',2' ,1': :10,9,11]estra-4,15-dien-3-one ;
4-[9,11α-dihydro-17β-hydroxy-3-oxo-17α-(1,1 ,2,2,2-pentafluoroethyl)-4 'H-naphth[3',2' ,1': 10,9, 11]estra-4, 15-dien-6' -yl]benzonitrile;
9,11α-dihydro-6'-(4-fluorophenyl)-17β-hydroxy-17α-(1,1,2,2,2-pentafluoroethyl)-4' H-naphth[3',2' ,1': :10,9,11]estra-4,15-dien-3-one ;
9, 11α-dihydro-17β-hydroxy-17α-(1,1,2,2,2-pentafluoroethyl)-6'-(3-pyridinyl)-4' H-naphth[3' ,2' ,1': :10,9,11] estra-4,15-dien-3-one ;
17β-hydroxy-11β-(4-hydroxyphenyl)-17α-(1,1,2,2,2-pentafluoroethyl)estra-4,9-dien-3-one;
17β-hydroxy-11β-(4-hydroxyphenyl)-17α-(1,1,2, 2, 2-pentafluoroethyl)estr-4-en-3-one;
9,11α-dihydro-6',17β-dihydroxy-17α-(1,1,2,2,2-pentafluoroethyl)-4'H-naphth[3',2', 1': 10,9,11]estr-4-en-3-one;
11β-[4-(acetyloxy)phenyl]-17β-hydroxy-17α-(1,1,2,2,2-pentafluoroethyl)estra-4,9-dien-3-one;
11β-[4-(acetyloxy)phenyl]-17β-hydroxy-17α-(1,1,2,2,2-pentafluoroethyl)estr-4-en-3-one
6'-acetyloxy-9,11α-dihydro-17β-hydroxy-17α-(1, 1,2,2,2-pentafluoroethyl)-4'H-naphth[3',2',1':10,9,11]estr-4-en-3-one;
17β-hydroxy-11β-[4-(hydroxymethyl)phenyl]-17α-(1,1,2,2,2-pentafluoroethyl)estra-4,9-dien-3-one;
17β-hydroxy-11β-[4-(hydroxymethyl)phenyl]-17α-(1,1,2,2,2-pentafluoroethyl)estr-4-en-3-one;
9,11α-dihydro-17β-hydroxy-6'-hydroxymethyl-17α-(1,1,2,2,2-pentafluoroethyl)-4' H-naphth[3' ,2' 1': 10,9, 11]estr-4-en-3-one;
4-[17β-hydroxy-3-oxo-17α-(1,1,2,2,2-pentafluoroethyl)estra-4,9-dien-11β-yl]benzaldehyde;
4-[17β-hydroxy-3-oxo-17α-(1,1,2,2,2-pentafluoroethyl)estr-4-en-11β-yl]benzaldehyde;
9,11 α-dihydro-17β-hydroxy-3-oxo-17α-(1,1,2,2,2-pentafluoroethyl)-4'H-naphth[3' ,2' 1': 10,9,11]estr-4-en-6' -al ;
4-[17β-hydroxy-3-oxo-17α-(1,1,2,2,2-pentafluoroethyl)estra-4,9-dien-11β-yl]benzoic acid methyl ester;
4-[17β-hydroxy-3-oxo-17α-(1,1,2,2,2-pentafluoroethyl)estr-4-en-11β-yl]benzoic acid methyl ester;
9, 11α-dihydro-17β-hydroxy-3-oxo-17α-(1,1,2,2,2-pentafluoroethyl)-4'H-naphth[3',2',1' 10,9, 11] estr-4-en-6'-carboxylic acid methyl ester;
17β-hydroxy-11 β-[4-(1-hydroxyethyl)phenyl]-17α-(1,1,2,2,2-pentafluoroethyl)estra-4,9-dien-3-one ;
17β-hydroxy-11β-[4-(1-hydroxyethyl)phenyl]-17α-(1,1,2,2,2-pentafluoroethyl)estr-4-en-3-one;
9,11α-dihydro-17β-hydroxy-6'-(1-hydroxyethyl)-17α-(1,1,2,2,2-pentafluoroethyl)-4' H-naphth[3',2' ,1': 10,9,11]estr-4-en-3-one.

Preferred is 11β-(4-acetylphenyl)-17β-hydroxy-17α-(1,1,2,2,2-pentafluoroethyl)estra-4,9-dien-3-one (Compound A).

The antigestagens can, for example, be applied locally, topically, enterally or parenterally.

For the preferred oral administration, particularly suitable are tablets, dragees, capsules, pills, suspensions or solutions which can be prepared in a conventional manner with additives and carriers used in pharmacy. For local or topical application, vaginal pessaries or percutaneous systems such as skin plasters can be used for example. For parenteral application, particularly suitable are solutions, preferably oily or aqueous solutions, as well as suspensions or emulsions. Ampules are convenient unit dosages.

According to a preferred mode of operation, the 17α- fluoralkylated progesterone receptor antagonist is typically administered over a period of 1 to 6 days, preferably 1 to 4 days, and more preferably on day(s) 1-3 after ovulation and/or the removal of oocytes. Typically, the antigestagen is administered over a period of 1 to 6 days, preferably 1-4 days, and more preferably 1-3 days after ovulation induction with e.g. a chorionic gonadotropin.

Surprisingly, according to the invention, it has been found that low doses of a 17α-fluoralkylated progesterone receptor antagonists are effective in the use disclosed herein. The 17α-fluoralkylated progesterone receptor antagonists are preferably administered to a human female subject in a daily dosage amount of up to 10 mg per subject, preferably 0.1 - 2 mg per subject, more preferably 0.1-1 mg per subject, and most preferably 0.1-0.7 mg per subject. For mammals in general, a daily dosage amount is typically 0.01-1 mg/kg, preferably 0.01-0.3 mg/kg, and more preferably 0.01-0.1 mg/kg. The daily dose of antigestagen can be administered as a single dose or as divided dosages throughout the day.

In a preferred embodiment according to the invention, the antigestagen is administered on a single day to a human subject in an amount of 0.1 - 2 mg/per subject, more preferably 0.1-1 mg/per subject, and most preferably 0.1-0.7 mg/per subject or to a mammal in an amount of 0.01-1 mg/kg, preferably 0.01-0.3 mg/kg, and more preferably 0.01-0.1 mg/kg. For example, on day 1, 2, 3, 4, 5 or 6 following ovulation, removal of oocytes, or administration of a chorionic gonadotropin, preferably day 1-3, and more preferably day 2.

For any particular 17α-fluoralkylated progesterone receptor antagonist, the most appropriate dose can be determined, for example, by evaluation of the potency to induce premature menstruation in advanced luteal phase of the human cycle as described in e.g. Herrmann, W., et al, 1982, *Comptes Rendus* 294:933. The use of antigestagens for delaying endometrial maturation has been previously described, for example, in U.S. Patent No. 4,764,513, EP 0219447B1, Hegele-Hartung et al., 1992, Endocrinology 131:2446-2460.

The compounds of this invention can be employed in admixture with conventional excipients, i.e., pharmaceutically acceptable organic or inorganic carrier substances suitable for, for example, parenteral, enthral (e.g., oral) or topical application which do not deleteriously react with the active compounds. Suitable pharmaceutically acceptable carriers include but are not limited to water, salt solutions, alcohols, gum arabic, vegetable oils, benzyl alcohols, polyethylene glycols, gelatine, carbohydrates such as lactose, amylase or starch, magnesium stearate, talc, silicic acid, viscous paraffin, perfume oil, fatty acid monoglycerides and diglycerides, pentaerythritol fatty acid esters, hydroxy methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds. They can also be combined, where desired, with other active agents, e.g., vitamins.

Sustained or directed release compositions can be formulated, e.g., liposomes or those wherein the active compound is protected with differentially degradable coatings, e.g., by microencapsulation, multiple coatings, etc. It is also possible to freeze-dry the new compounds and use the lyophilizates obtained, for example, for the preparation of products for injection.

For topical application, there are employed as nonsprayable forms, viscous to semi-solid or solid forms comprising a carrier compatible with topical application and having a dynamic viscosity preferably greater than water. Suitable formulations include but are not limited to solutions, suspensions, emulsions, creams, ointments, powders, liniments, salves, aerosols, etc., which are, if desired, sterilized or mixed with auxiliary agents, e.g., preservatives, stabilizers, wetting agents, buffers or salts for influencing osmotic pressure, etc. For topical application, also suitable are sprayable aerosol preparations wherein the active ingredient, preferably in combination with a solid or liquid inert carrier material, is packaged in a squeeze bottle or in admixture with a pressurized volatile, normally gaseous propellant, e.g., a freon.

It will be appreciated that the actual preferred amounts of active compound in a specific case will vary according to the specific compound being utilized, the particular compositions formulated, the mode of application, and the particular situs and organism being treated. Dosages for a given host can be determined using conventional considerations, e.g., by customary comparison of the differential activities of the subject compounds of a known agent, e.g., by means of an appropriate, conventional pharmacological protocol.

### BRIEF DESCRIPTION OF THE FIGURES

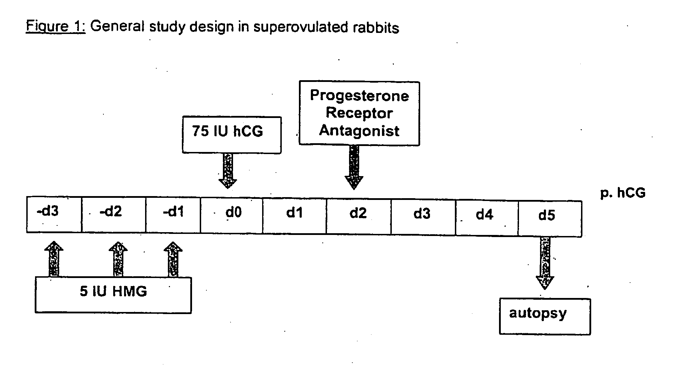

Figure 1 is a schematic illustration of the experiment described in Example 1. The day of administration of human chorionic gonadotropin (hCG) was designated as day 0 of pseudopregnancy (d0). As shown in the diagram, ovarian stimulation of the animals occurred on -d3, -d2 and -d1 of pseudpregnancy induction and the progesterone receptor antagonist was administered on d2 of psuedopregnancy.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius; and, unless otherwise indicated, all parts and percentages are by weight.

### EXAMPLES

### Example 1: Improvement of endometrial receptivity with low doses of a 17α-fluoralkylated progesterone receptor antagonist in the superovulated rabbit model.

The effects of a 17α-fluoralkylated progesterone receptor antagonist on endometrial activity was evaluated in the superovulated pseudopregnant rabbit model. The pseudopregnancy of the rabbit has proven to be a model system for the human luteal phase (Beier and Kuhnel, 1973, Hormone Res. 4:1-27; Fischer et al., 1985, Fertilitat 1:101-109. In this species, ovarian stimulation induces an advancement of secretory transformation (Delbos-Winter et al., 1987, Fertilitat 3: 87-93) as well as an enhancement of gland formation.

A total of 25 sexually mature nulliparous New Zealand White rabbits with a body weight of 3.1 to 4.0 kg were used for the analysis. The animals were divided into two control groups (groups 1 and 2) and three treatment groups (groups 3-5), with five animals in each group.

Control group 1 received 75 international units (IU) of human chorionic gonadotropin (hCG) administered intravenously in order to induce pseudopregnancy. The day of hCG-injection was designated as day 0 of pseudopregnancy (d0 p.hCG).

Control group 2 and treatment groups 3, 4, and 5 were gonadotropin-stimulated with 5 IU of the human menopausal gonadotropin (HMG) Pergonal^{®} (Serono Pharma, Unterschleilßheim, Germany) for 3 days, by subcutaneous injection, in order to induce multiple follicular growth. Pseudopregnancy was induced by the administration of human chorionic gonadotropin (hCG) as described for control group 1 above.

Treatment groups 3, 4 and 5 also received a single per oral application of a 17α -fluoralkylated progesterone receptor antagonist (Compound A) two days after pseudopregnancy induction with the administration of human chorionic gonadotropin (d2 p.hCG). The detailed treatment schedule is shown in Figure 1.

Animals in all treatment groups were sacrificed on day 5 following pseudopregnancy induction (d5 p.hCG), and uteri were removed. Relative uterine wet weights were determined (in mg/100 g body weight) and a part of the uterus was snap frozen in liquid nitrogen for determination of uteroglobin expression by in situ hybridization. Uteroglobin expression is known as a highly sensitive parameter for determination of endometrial receptivity (Beier, H.M., 1968, Biochem Biophys. Acta.160:289-291 ; Beier, H.M., 1982, in: Beier, H.M. and Karlson, P. (eds.), Proteins and Steroids in Early Pregnancy), and is a marker for secretory activity (Beier, 1976, J. Reprod. Fertil. Suppl. 25:53-69) and differentiation of the endometrial epithelium (Krusche & Beier, 1994, Ann. Anat. 176:23-31). In situ hybridization for detection of uteroglobin expression was conducted as previously described in Krusche & Beier, above. Another piece of the uterus was removed and processed for paraffin histology in order to determine the endometrial transformation status using the McPhail-Index (McPhail Mk, 1934, J. Phys. 83:145-156) The McPhail Index reflects different degrees of progestogenic (transforming) activity in the rabbit endometrium:
1. no transformation
2. low transformation
3. pronounced transformation
4. high transformation
The results are presented in Table 1 below.

**Table 1**

| Effect of a single treatment of a 17α-fluoralkylated progesterone receptor antagonist on uterus weight, endometrial transformation, and uteroglobin mRNA expression at five days after pseudopregnancy induction (d5 p.hCG) in the superovulated pseudopregnant rabbit. | | | | | | |
|---|---|---|---|---|---|---|
| Group | Treatment Schedule | | | Measurement endpoints at d5 p. hCG | | |
| | -d3, -d2, -d1 p.hCG | d0 p.hCG | d2 p.hCG | | | |
| | 5 IU HMG | 75 IU HCG | Progesterone receptor antagonist [mg/kg] | Uterine wet weight [mg/kg] [x±SD] | Mc-Phail [x±SD] | Uteroglobin ISH [expression in luminal epithelial cells] |
| 1 | - | + | - | 324 ±99 | 3.2 ±0.2 | very high expression |
| 2 | + | + | 0 | 448 ±80 | 3.9 ±0.1 | very low expression |
| 3 | + | + | 0.1 | 305 ±48 | 3.0 ±0.1 | very high expression |
| 4 | + | + | 1 | 227 ±56 | 1.5 ±0.3 | very low expression |
| 5 | + | + | 10 | 144 ±30 | 1.1 ±0.3 | absent |

As shown in the table, animals which were subject to ovarian hyperstimulation, but which were not treated with an antigestagen (Group 2), showed advanced endometrial maturation as demonstrated by nearly indetectable uteroglobin expression in endometrial luminal epithelial cells, the weight of the uterus, and the relatively high Mc-Phail index score. However, animals which were subject to ovarian hyperstimulation and which were treated with an antigestagen, Groups 3-5, showed a delay in endometrial maturation, as demonstrated by the uterine wet weights and the Mc-Phail index scores. For animals which were treated with a low dose of progesterone receptor antagonist, 0.1 mg/kg (Group 3), endometrial maturation roughly corresponded with that of control animals which were not subject to ovarian hyperstimulation (Group 1). In contrast, higher doses of progesterone receptor antagonist, 1 mg/kg (group 4) and 10 mg/kg (group 5), apparently counteracted the advancement of endometrium maturation associated with ovarian hyperstimulation and also delayed the timing of maturation beyond that observed in animals which were not subject to hyperovarian stimulation (Group 1).

The preceding examples can be repeated with similar success by substituting the generically or specifically described reactants and/or operating conditions of this invention for those used in the preceding examples.

## Claims

1. Use of at least one compound of formula I 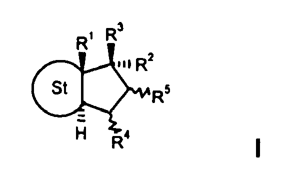 wherein
R¹ is methyl or ethyl,
R² is CₙFₘHₒ, wherein n is 1-6, preferably 2, 3, 4, 5 or 6, m > 1 and m+o=2n+1,
R³ is a free, etherified or esterified hydroxyl group,
R⁴ and R⁵ each is hydrogen, or together form and additional bond or a methylene group,
St is a steroidal ABC-ring system of partial formula A, B or C 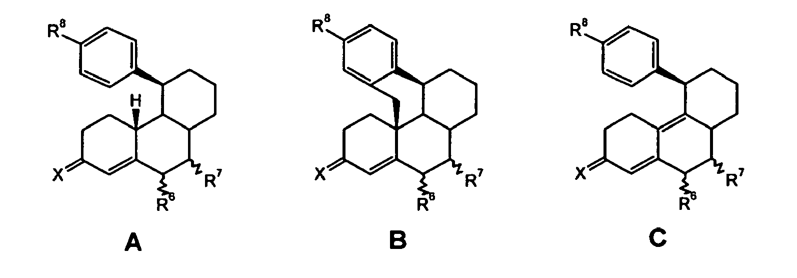
wherein
R⁶ is hydrogen, a straight-chain C₁-C₄ alkyl group or branched C₃-C₄ alkyl group or halogen,
R⁷ is hydrogen, a straight-chain C₁-C₄ alkyl group or a branched C₃-C₄ alkyl group, or
if St is a steroidal ABC-ring system A or B, in addition
R⁶ and R⁷ together can form an additional bond,
X is oxygen, hydroxyimino (=N-OH) or two hydrogen atoms,
R⁸ is Y or aryl that is optionally substituted in several places with a group Y, other than H,
Y is hydrogen, halogen, -OH, -NO₂, -N₃, -CN, -NR^{9a}R⁹b, -NHSO₂R⁹, -CO₂R⁹, C₁-C₁₀ alkyl, C₁-C₁₀ alkoxy, C₁C₁₀ alkanoyloxy, benzoyloxy, C₁-C₁₀ alkanoyl, C₁-C₁₀ hydroxyalkyl or benzoyl,
R^{9a} and R^{9b} are the same or different and each is hydrogen or C₁-C₁₀ alkyl,
R⁹ is hydrogen or C₁-C₁₀alkyl,
and for -NR^{9a}R^{9b} radicals,as well as their physiologically compatible salts with acids and for-CO₂R⁹ radicals with R⁹ being hydrogen, as well as their physiologically compatible salts with bases, for production of a medicament for inhibiting the occurrence of advanced endometrium maturation in a human female subject undergoing fertility enhancing treatment.

2. Use according to claim 1, wherein the compound of formula I is administered to the female subject during the post-ovulatory phase of the endometrial cycle.

3. Use according claim 2, wherein the compound of formula I is used in a daily dosage amount of 0.1 - 2 mg per subject.

4. Use according claim 3, wherein the fertility treatment comprises the administration to the subject of a follicle stimulating agent comprising follicle stimulating hormone.

5. Use according to claim 3, wherein the compound of formula I is used in an amount of 0.1-2 mg per subject on a single day during the post-ovulatory phase of the endometrial cycle.

6. Use according to claim 5, wherein the compound of formula I is administered orally.

## Patentansprüche

1. Verwendung wenigstens einer Verbindung der Formel I 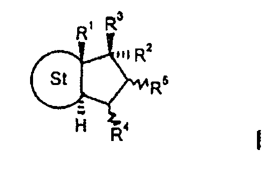 wobei
R¹ für Methyl oder Ethyl steht,
R₂ für CₙFₘHₒ steht, wobei n für 1-6, vorzugweise 2,3,4,5 oder 6, m > 1 und m + o = 2n + 1 steht,
R³ für eine freie, veretherte oder veresterte Hydroxylgruppe steht,
R⁴ und R⁵ jeweils für Wasserstoff stehen oder zusammen eine zusätzliche Bindung oder eine Methylengruppe bilden,
St für ein steroidales ABC-Ringsystem der Teilformel A, B, oder C steht 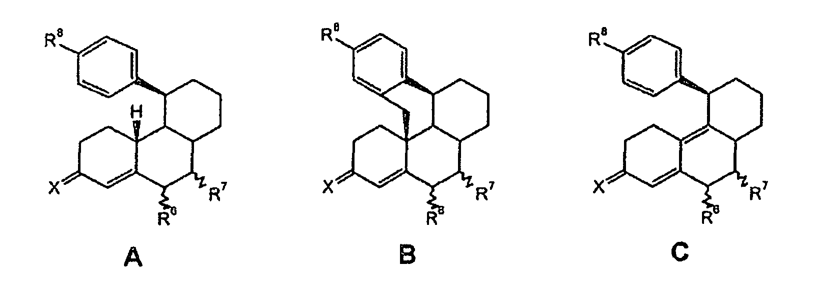
wobei
R⁶ für Wasserstoff, eine gradkettige C₁-C₄-Alkylgruppe oder ein verzweigte C₃-C₄-Alkylgruppe oder Halogen steht,
R⁷ für Wasserstoff, eine gradkettige C₁-C₄-Alkylgruppe oder eine verzweigte C₃-C₄-Alkylgruppe steht, oder,
wenn St für ein steroidales ABC-Ringsystem der Teilformel A oder B steht, zusätzlich
R⁶ und R⁷ zusammen eine zusätzliche Bindung bilden können,
X für Sauerstoff, Hydroxyimino (=N-OH) oder zwei Wasserstoffatome steht,
R⁸ für Y oder gegebenenfalls in mehreren Positionen durch eine Gruppe Y, bei der es sich nicht um H handelt, substituiertes Aryl steht,
Y für Wasserstoff, Halogen, -OH, -NO₂, N₃, -CN, -NR^{9a}R⁹b, -NHSO₂R⁹, -CO₂R⁹, C₁-C₁₀-Alkyl, C₁-C₁₀-Alkoxy, C₁-C₁₀-Alkanoyloxy, Benzoyloxy, C₁-C₁₀-Alkanoyl, C₁-C₁₀-Hydroxyalkyl oder Benzoyl steht,
R^{9a} und R^{9b} gleich oder verschieden sind und jeweils für Wasserstoff oder C₁-C₁₀-Alkyl stehen,
R⁹ für Wasserstoff oder C₁-C₁₀-Alkyl steht,
und bei -NR^{9a}R^{9b}-Resten auch deren physiologisch verträglicher Salze mit Säuren und bei -CO₂R⁹-Resten, in denen R⁹ für Wasserstoff steht, auch deren physiologisch verträglicher Salze mit Basen, zur Herstellung eines Medikaments zur Inhibierung des Einsetzens von fortgeschrittener Endometriumreifung bei einer humanen Patientin, die sich einer Fruchtbarkeitsbehandlung unterzieht.

2. Verwendung nach Anspruch 1, wobei man der Patientin die Verbindung der Formel I während der postovulatorischen Phase des Endometriumzyklus verabreicht.

3. Verwendung nach Anspruch 2, wobei die Verbindung der Formel I in einer Tagesdosismenge von 0,1-2 mg pro Patientin angewendet wird.

4. Verwendung nach Anspruch 3, wobei die Fruchtbarkeitsbehandlung die Verabreichung eines follikelstimulierendes Hormon enthaltenden follikelstimulierenden Mittels an die Patientin umfaßt.

5. Verwendung nach Anspruch 3, wobei die Verbindung der Formel I in einer Menge von 0,1-2 mg pro Patientin an einem einzelnen Tag während der postovulatorischen Phase des Endometriumzyklus angewendet wird.

6. Verwendung nach Anspruch 5, wobei die Verbindung der Formel I oral verabreicht wird.

## Revendications

1. Utilisation d'au moins un composé de formule I : 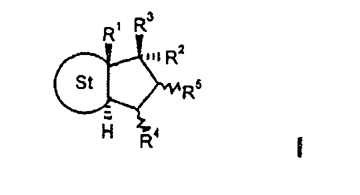 dans laquelle :
R¹ est un groupe méthyle ou un groupe éthyle,
R² est un groupe CₙFₘHₒ, dans lequel n vaut de 1 à 6, de préférence 2, 3, 4, 5 ou 6, m > 1 et m + o = 2n + 1,
R³ est un groupe hydroxyle libre, éthérifié ou estérifié,
R⁴ et R⁵ sont chacun un atome d'hydrogène, ou forment conjointement une liaison supplémentaire ou un groupe méthylène,
St est un système de noyau ABC stéroïdien de formules partielles A, B ou C 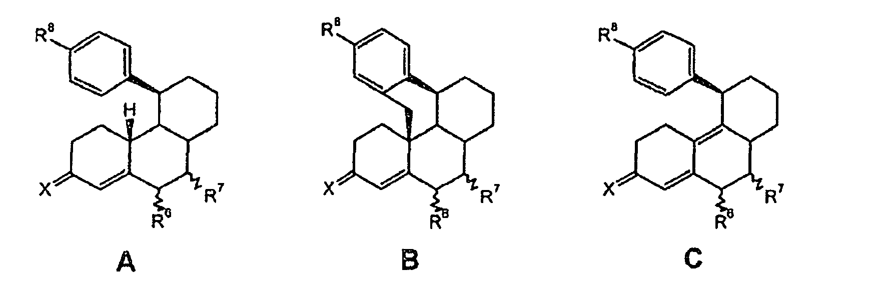
dans lesquelles :
R⁶ est un atome d'hydrogène, un groupe alkyle en C₁-C₄ à chaîne linéaire ou un groupe alkyle en C₃-C₄ ramifié, ou un atome d'halogène,
R⁷ est un atome d'hydrogène, ou un groupe alkyle en C₁-C₄ à chaîne linéaire ou un groupe alkyle en C₃-C₄ ramifié, ou
si St est un système de noyau ABC stéroïdien A ou B, en outre
R⁶ et R⁷ peuvent former conjointement une liaison supplémentaire,
X est un atome d'oxygène, un groupe hydroxyimino (= N-OH) ou deux atomes d'hydrogène,
R⁸ est un groupe Y ou un groupe aryle qui est éventuellement substitué en plusieurs endroits par un groupe Y, autre qu'un atome d'hydrogène,
Y est un atome d'hydrogène, un atome d'halogène, un groupe -OH, un groupe -NO₂, un groupe -N₃, un groupe -CN, un groupe -NR^{9a}R^{9b}, un groupe -NHSO₂R⁹ , un groupe -CO₂R⁹, un groupe alkyle en C₁-C₁₀, un groupe alcoxy en C₁-C₁₀, un groupe alcanoyloxy en C₁-C₁₀, un groupe benzoyloxy, un groupe alcanoyle en C₁-C₁₀, un groupe hydroxyalkyle en C₁-C₁₀ ou un groupe benzoyle,
R^{9a} R^{9b} et sont identiques ou différents et chacun est un atome d'hydrogène ou un groupe alkyle en C₁-C₁₀,
R⁹ est un atome d'hydrogène ou un groupe alkyle en C₁-C₁₀,
et pour des radicaux -NR^{9a}R^{9b}, ainsi que leurs sels physiologiquement compatibles avec des acides, et pour des radicaux -CO₂R⁹, dans lesquels R⁹ est un atome d'hydrogène, ainsi que leurs sels physiologiquement compatibles avec des bases, pour la production d'un médicament destiné à l'inhibition de l'apparition d'une maturation avancée de l'endomètre chez une femme subissant un traitement stimulant la fertilité.

2. Utilisation selon la revendication 1, dans laquelle le composé de formule I est administré à la femme durant la phase post-ovulatoire du cycle endométrial.

3. Utilisation selon la revendication 2, dans laquelle le composé de formule I est utilisé en une quantité de dosage journalier de 0,1 à 2 mg par sujet.

4. Utilisation selon la revendication 3, dans laquelle le traitement de fertilité comprend l'administration au sujet d'un agent stimulant les follicules comprenant de la follicostimuline.

5. Utilisation selon la revendication 3, dans laquelle le composé de formule I est utilisé en une quantité de 0,1 à 2 mg par sujet, sur un seul jour durant la phase post-ovulatoire du cycle endométrial.

6. Utilisation selon la revendication 5, dans laquelle le composé de formule I est administré par voie orale.
